(19) Europäisches Patentamt
European Patent Office
Office européen des brevets

(11) Veröffentlichungsnummer: **0 303 951 B1**

(12) # EUROPÄISCHE PATENTSCHRIFT

(45) Veröffentlichungstag der Patentschrift: **04.11.92**

(51) Int. Cl.[5]: **A61K 31/505**

(21) Anmeldenummer: **88112965.4**

(22) Anmeldetag: **10.08.88**

(54) **2-Pyrimidinyl-1-piperazin-Derivate zur Behandlung von Alkoholismus.**

(30) Priorität: **21.08.87 DE 3727879**

(43) Veröffentlichungstag der Anmeldung:
**22.02.89 Patentblatt 89/08**

(45) Bekanntmachung des Hinweises auf die Patenterteilung:
**04.11.92 Patentblatt 92/45**

(84) Benannte Vertragsstaaten:
**AT BE CH DE ES FR GB GR IT LI LU NL SE**

(56) Entgegenhaltungen:

**JOURNAL OF STUDIES ON ALCOHOL, Band 47, Nr. 4, 1986, Seiten 269-273; R.E. MEYER et al.: "Anxiolytics and the alcoholic patient"**

**DRUG AND ALCOHOL DEPENDENCE, Band 21, Nr. 2, Mai 1988, Seiten 99-104, Elsevier Scientific Publishers Ireland Ltd, IE; K. OPITZ et al.: "Volitional oral intake of nicotine in tupaias: drug-induced alterations"**

**PSYCHOPATHOLOGY, Band 22, (Ergänz. 1), 1989, Seiten 13-20, S. Karger AG, Basel, CH; M.S. ELISON: "The new generation of serotonergic anxiolytics: possible clinical roles"**

**PSYCHOPHARMACOLOGY, Band 89, Nr. 4, 1986, Seite 55, Zusammenfassung Nr. 166; I. LUCKI: "The nonbenzodlazepine anxiolytics buspirone and isapirone antagonize serotonin-mediated behavioral responses"**

**JOURNAL OF CLINICAL PSYCHOPHARMACOLOGY, Band 7, Nr. 1, Februar 1987, Seiten 30-33, Williams & Wilkins Co., Baltimore, US; I. CSANALOSI et al.: "Gepirone in anxiety: a pilot study"**

(73) Patentinhaber: **Troponwerke GmbH & Co. KG**
**Berliner Strasse 156**
**W-5000 Köln 80(DE)**

(72) Erfinder: **Traber, Jörg, Dr.**
**Löwenburgstrasse 12**
**W-5204 Lohmar 21(DE)**
Erfinder: **Opitz, Klaus, Prof., Dr.**
**Goerlitzer Strasse 102**
**W-4400 Muenster(DE)**

(74) Vertreter: **Mann, Volker, Dr. et al**
**c/o Bayer AG Konzernverwaltung RP Patentabteilung**
**W-5090 Leverkusen 1, Bayerwerk(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft die Verwendung von 2-Pyrimidinyl-1-piperazin-Derivatenzur Herstellung von Arzneimitteln für die Behandlung von Alkoholismus und entsprechende Arzneimittel.

Aus der EP-A 0 129 128 sind 2-Pyrimidinyl-1-piperazin-Derivate und ihre im wesentlichen anxiolytische Wirkung bekannt. Bekannte Wirkstoffe aus dieser Substanzklasse sind 8-[4-N-[4-(2-Pyrimidinyl)-1-piperazinyl]butyl]-8-azaspiro[4,5]-decan-7,9-dion-hydrochlorid (nach INN: Buspiron, Pharmacol. Biochem. Behav. 23, 687 bis 694 (1985)), 4,4-Dimethyl-1-[4-[4-(2-pyrimidinyl)-1-piperazinyl]butyl]-2,6-piperidindion-hydrochlorid (nach INN: Gepiron, Naunyn-Schmiedeberg's Arch. Pharmacol. 335, 454 bis 464 (1987)) und 2-(4-(4-(2-Pyrimidinyl)-1-piperazinyl)-butyl)-1,2-benzoisothiazol-3(2H)-on-1,1-dioxidhydrochlorid (nach INN: Ipsapiron, Naunyn-Schmiedeberg's Arch. Pharmacol. 328, 467 bis 470 (1985)).

Aus Alcohol 4 (1), 49 bis 56 (1987) ist bekannt, daß Affen mit einer Präferenz für Alkohol in der Zeit einer Behandlung mit Buspiron eine geringere Präferenz für Alkohol zeigen.

Es wurde die Verwendung von 2-Pyrimidinyl-1-piperazin-Derivaten der Formel

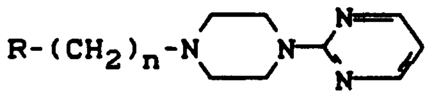

(I)

in der

n für eine der Zahlen 2, 3, 4, 5 oder 6 steht und

R für einen der Reste

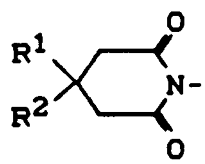 oder 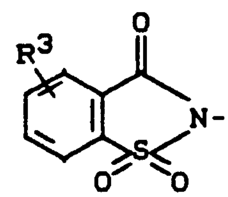 steht

worin

$R^1$, $R^2$ und $R^3$ gleich oder verschieden sind und Wasserstoff oder Niederalkyl bedeuten

und/oder deren Salze

zur Herstellung von Arzneimitteln zur Behandlung von Alkoholismus gefunden.

Entsprechende Arzneimittel sind gekennzeichnet durch einen Gehalt an 2-Pyrimidinyl-1-piperazin-Derivaten der Formel

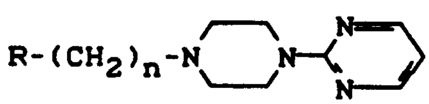

(I)

in der

n für eine der Zahlen 2, 3, 4, 5 oder 6 steht und

R für einen der Reste

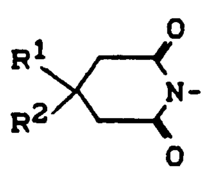 oder 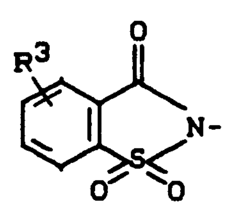 steht

worin

$R^1$, $R^2$ und $R^3$ gleich oder verschieden sind und Wasserstoff oder Niederalkyl bedeuten

und/oder deren Salze.

Überraschenderweise zeigen die 2-Pyrimidinyl-1-piperazin-Derivate eine gegenüber Buspiron überlegene Wirkung bei der Behandlung von Alkoholismus.

Im Rahmen der Formel (I) bedeutet Niederalkyl im allgemeinen einen geradkettigen oder verzweigten Kohlenwasserstoffrest mit 1 bis etwa 6 Kohlenstoffatomen. Beispielsweise sei Methyl, Ethyl, Propyl, Isopropyl, Butyl, Isobutyl, Pentyl, Isopentyl, Hexyl und Isohexyl genannt. Bevorzugt werden Methyl und Ethyl.

Bevorzugt werden 2-Pyrimidinyl-1-piperazin-Derivate der Formel (I), wobei

n für eine der Zahlen 3 oder 4 steht und

$R^1$, $R^2$ und $R^3$ Wasserstoff oder Methyl bedeuten.

Als Salze seien pharmakologisch unbedenkliche Salze, wie die Hydrochloride, genannt.

Insbesondere bevorzugt werden Ipsapiron und Gepiron.

Die Herstellung der 2-Pyrimidinyl-1-piperazin-Derivate ist an sich bekannt (DE-A 33 21 969) und kann beispielsweise durch Umsetzung von entsprechenden Benzisothiazolen mit (Piperazinyl)-pyrimidinen erfolgen.

Die Arzneimittel enthalten im allgemeinen 1 bis 15 Gew.-%, bevorzugt 5 bis 10 Gew.-% an 2-Pyrimidinyl-1-piperazin-Derivaten.

Es ist selbstverständlich möglich, daß die Arzneimittel weitere an sich bekannte Wirkstoffe enthalten.

Die Arzneimittel können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Falle der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B. Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylzellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden. Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Ein brauchbares Mittel zur medikamentösen Behandlung des Alkoholismus ist nicht bekannt. Die Enzymhemmstoffe Disulfiram und Nitrefazol bewirken eine unangenehme Reaktion, wenn der damit behandelte Alkoholiker trotz Verbots trinkt.

Im Gegensatz zu diesen Substanzen hemmen die 2-Pyrimidinyl-1-piperazin-Derivate den freiwilligen Alkoholkonsum Alkoholabhängiger. Insbesondere kann der Gebrauch von 2-Pyrimidinyl-1-piperazin-Derivaten das Rückfälligwerden verhindern.

Beispiel 1

Darstellung von 2-(4-(4-(2-Pyrimidinyl)-1-piperazinyl)-butyl)-1,2-benzoisothiazol-3(2H)-on-1,1-dioxid

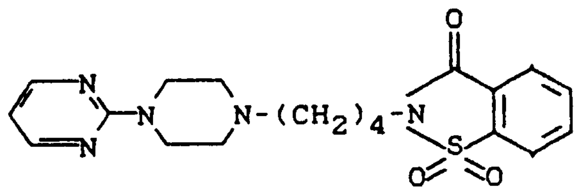

0,02 Mol 2-(4-Brombutyl)-1,2-benzoisothiazol-3-(2H)-on-1,1-dioxid und 0,02 Mol 1-(2-Pyrimidyl)-piperazin werden mit 0,02 Mol $K_2CO_3$ in 150 ml absolutem Dimethylformamid (DMF) 1 Stunde bei 100 °C gerührt. Danach engt man ein. Mit Wasser versetzt, wird die organische Substanz in Methylenchlorid ($CH_2Cl_2$) aufgenommen. Die getrocknete $CH_2Cl_2$-Phase wird auf eine Kieselgelsäule gegeben und mit $CH_2Cl_2/CH_3OH$ (95:5) eluiert.

Ausbeute: 34 % der Theorie; Fp.: 138-139 °C.

Beispiel 2

Bestimmung der Wirksamkeit

Ethanol-präferente Ratten werden unter standardisierten Bedingungen (12-Stunden-Hell-Dunkel-Rhythmus, 23 ± 1 °C) einzeln in großen Makrolonkäfigen gehalten. Den Tieren stehen Zuchtfutter, Trinkwasser und Ethanol 10 Vol-% in unbeschränkten Mengen zur Verfügung, jedoch nur während der Dunkelphase von 20.00 bis 08.00 Uhr. Die zu untersuchenden Substanzen werden einmalig oral zugeführt (2 ml/kg, Schlundsonde), und zwar 30-20 min vor Beginn der Dunkelphase. Die Futtergefäße und die Trinkflaschen werden jeden Morgen gewogen und die verbrauchten Mengen festgestellt. Als Maß der Präferenz gilt die getrunkene Alkoholmenge (10 Vol-%) im Prozent der Gesamtflüssigkeitsaufnahme. Die nach Verabreichung einer Prüfsubstanz gemessenen Verbräuche werden mit den mittleren Verbräuchen an den drei voraufgegangenen Tagen (Vorperiode) verglichen. Angegeben in Tabelle 1 ist die jeweilige Änderung der Gesamtflüssigkeitsaufnahme und des relativen Alkoholkonsums (10 Vol.-% Ethanol) in Prozent der während der jeweils dreitägigen Vorperiode ermittelten Durchschnittswerte. Statistische Berechnungen erfolgen nach dem Student's t-Test für gepaarte Werte.

Tabelle 1

| Ingestives Verhalten von acht männlichen ethanolpräferenten Ratten | | | |
|---|---|---|---|
| Substanz | Dosis (mg/kg p.o.) | Änderung der Gesamtflüssigkeitsaufnahme (%) | Änderung des relativen Alkoholkonsums (%) |
| Ipsapiron | 20 | + 30,9*** | - 43,1*** |
| Gepiron | 20 | + 15,3** | - 49,8*** |
| Buspiron | 20 | + 1,6 ns | - 1,6 ns |

** p <0,01
*** p <0,001

Die Daten zeigen für Ipsapiron und für Gepiron eine deutliche Zunahme der Gesamtflüssigkeitsaufnahme, die jedoch von einer starken, hochsignifikanten Abnahme des relativen Alkoholkonsums begleitet wird. Demgemäß nimmt nach Gabe von Ipsapiron oder Gepiron die Präferenz der Ratten für Alkohol stark ab. Buspiron zeigt keinen signifikanten Effekt.

**Patentansprüche**

1. Verwendung von 2-Pyrimidinyl-1-piperazin-Derivaten der Formel

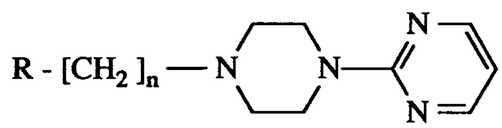

in der

| | |
|---|---|
| n | -für eine der Zahlen 2,3,4,5 oder 6 steht, und |
| R | -für einen der Reste |

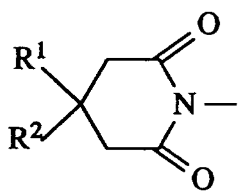

oder

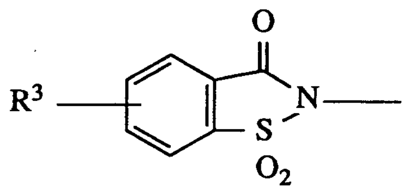

steht,
wobei

| | |
|---|---|
| $R^1$,$R^2$ und $R^3$ | gleich oder verschieden sind und Wasserstoff oder Niederalkyl bedeuten, |

und deren Salze
zur Herstellung von Arzneimitteln zur Behandlung von Alkoholismus.

2. Verwendung nach Anspruch 1, dadurch gekennzeichnet, daß 2-Pyrimidinyl-1-piperazin-Derivate der Formel nach Anspruch 1, worin

| | |
|---|---|
| n | -für eine Zahl oder 4 steht, und |
| $R^1$, $R^2$ und $R^3$ | für Wasserstoff oder Methyl stehen, |

einsetzt.

3. Verwendung nach Anspruch 1, dadurch gekennzeichnet, daß als 2-Pyrimidinyl-1-piperazin-Derivat Gepiron eingesetzt wird.

4. Verwendung nach Anspruch 1, dadurch gekennzeichnet, daß als 2-Pyrimidinyl-1-piperazin-Derivat Ipsapiron eingesetzt wird.

## Claims

1. Use of 2-pyrimidinyl-1-piperazine derivatives of the formula

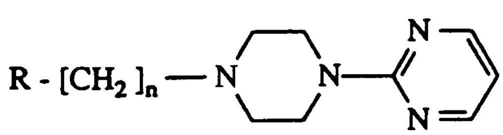

in which

| | |
|---|---|
| n | - represents one of the numbers 2, 3, 4, 5 or 6, and |
| R | - represents one of the radicals |

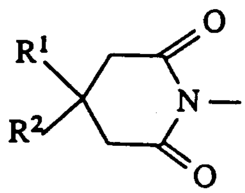

or

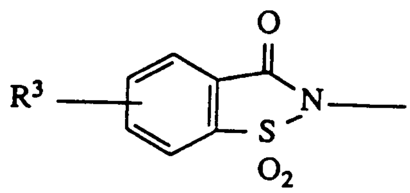

in which

R', $R^2$ and $R^3$ are identical or different and denote hydrogen or lower alkyl,

and their salts

for the production of medicaments for the treatment of alcoholism.

**2.** Use according to Claim 1, characterised in that 2-pyrimidinyl-1-piperazine derivatives of the formula according to Claim 1 are employed, in which

n - represents a number 3 or 4, and

R', $R^2$ and $R^3$ represent hydrogen or methyl.

**3.** Use according to Claim 1, characterised in that gepirone is employed as the 2-pyrimidinyl-1-piperazine derivative.

**4.** Use according to Claim 1, characterised in that ipsapirone is employed as the 2-pyrimidinyl-1-piperazine derivative.

## Revendications

**1.** Utilisation de dérivés de la 2-pyrimidinyl-1-pipérazine de formule :

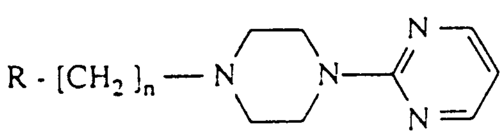

dans laquelle

n est égal à 2, 3, 4, 5 ou 6 et

R représente l'un des groupes

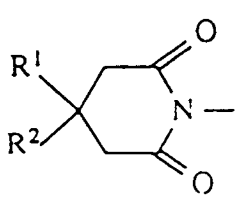

ou

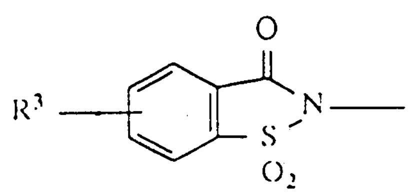

dans lesquels

$R^1$, $R^2$ et $R^3$, ayant des significations identiques ou différentes, représentant chacun l'hydrogène ou un groupe alkyle inférieur, et de leurs sels

pour la préparation de médicaments pour le traitement de l'alcoolisme.

**2.** Utilisation selon la revendication 1, caractérisée en ce que l'on utilise des dérivés de la 2-pyrimidinyl-1-pipérazine de formule donnée dans la revendication 1,

dans laquelle

n est égal à 3 ou 4 et

$R^1$, $R^2$ et $R^3$ représentent chacun l'hydrogène ou un groupe méthyle.

**3.** Utilisation selon la revendication 1, caractérisée en ce que le dérivé de 2-pyrimidinyl-1-pipérazine est le Gepiron.

**4.** Utilisation selon la revendication 1, caractérisée en ce que le dérivé de 2-pyrimidinyl-1-pipérazine est l'Ipsapiron.